# EUROPÄISCHE PATENTANMELDUNG

(11) **EP 3 360 539 A1**
(43) Veröffentlichungstag der Anmeldung: **15.08.2018**
(21) Anmeldenummer: 17155355.5
(22) Anmeldetag: 09.02.2017
(51) Int. Cl.: A61K 9/08, A61K 47/40, A61K 31/4178

(54) **PHARMAZEUTISCHE FORMULIERUNG**

(71) Anmelder: SapioTec GmbH, 97082 Würzburg (DE)
(72) Erfinder: ROEWER, Norbert, 97082 Würzburg (DE); BROSCHEIT, Jens, 97209 Würzburg (DE)
(74) Vertreter: Glawe, Delfs, Moll

(57) **Zusammenfassung**

Gegenstand der Erfindung ist eine pharmazeutische Formulierung umfassend einen Komplex aus Dantrolen oder dessen Salz und HPBCD, dadurch gekennzeichnet, dass der pH-Wert der Formulierung zwischen 8,5 und 10,5 liegt.

## Beschreibung

Die Erfindung betrifft eine pharmazeutische Formulierung, eine gebrauchsfertige Injektionslösung zur intravenösen Administration und ein Verfahren zum Herstellen der Formulierung.

In WO 2010/126818 A1 wird eine bevorzugte intranasale Administration von Dantrolen und dessen Salzen beschrieben. Dabei offenbart das Dokument das Mischen und Lösen von einem Überschuss an Dantrolen-Natrium mit 2-Hydroxypropyl-β-cyclodextrin (HPBCD) bei einem pH-Wert von 8.

In der Veröffentlichung von Fuchs et al., Brain Penetration of WEB 2086 (Apafant) and Dantrolen in Mdr1a (P-Glycoprotein) and Bcrp Knockout Rats, Drug Metabolism and Diposition, Oktober 2014, Vol. 42, Nr. 10, 1761-1765, werden Versuche beschrieben, in denen Dantrolen als Wirkstoffverbindung in HPBCD (10%) gelöst wird. Die Konzentration des gelösten Dantrolens in Lösung beträgt 0,137 mg/ml und die erhaltene Lösung wird intravenös injiziert.

Der Erfindung liegt die Aufgabe zu Grunde, eine pharmazeutische Formulierung zu schaffen, die eine verbesserte Stabilität und Löslichkeit von Dantrolen oder dessen Salz aufweist und somit die Zurverfügungstellung einer gebrauchsfertige Injektionslösung für eine einfachere und schnellere intravenöse Administration ermöglicht.

Gelöst wird diese Aufgabe durch eine pharmazeutische Formulierung gemäß dem Hauptanspruch. Die Erfindung betrifft somit insbesondere eine pharmazeutische Formulierung umfassend einen Komplex aus Dantrolen oder dessen Salz und HPBCD, dadurch gekennzeichnet, dass der pH-Wert der Formulierung zwischen 8,5 und 10,5 liegt.

Zunächst sein einige im Rahmen der Erfindung verwendete Begriffe erläutert.

Dantrolen ist ein Hydantoin-Derivat aus der Gruppe der Muskelrelaxantien und wird als Arzneistoff, oral in Kapselform zur Behandlung spastischer Syndrome mit krankhaft gesteigerter Muskelspannung und intravenös meist bei der malignen Hyperthermie und beim malignen neuroleptischen Syndrom eingesetzt. Problematisch sind die schlechte Wasserlöslichkeit von Dantrolen und die daraus entstehende Schwierigkeit, in Notsituationen schnell und stabile Lösungen zur intravenösen Anwendung herzustellen.

Die maligne Hyperthermie ist eine seltene, aber lebensbedrohende Narkosekomplikation. Triggersubstanzen sind vor allem volatile Inhalationsanästhetika und depolarisierende Muskelrelaxtatien. Die Symptomatik ist sehr variabel und umfasst Muskelstarre, Herzrasen, eine erhöhte Produktion von Kohlenstoffdioxid und Temperaturerhöhung bis hin zur Übersäuerung des Körpers sowie Stoffwechsel- und Organversagen, die letztlich zum Tod führen können. Durch sofortige Unterbrechung der Narkosemittel-Zufuhr und Behandlung mit dem Wirkstoff Dantrolen kann eine maligne Hyperthermie wirksam behandelt werden. Durch die Einführung des Wirkstoffes Dantrolen konnte in der Vergangenheit die durch solche Komplikationen auftretende Sterblichkeit gesenkt werden.

Das maligne neuroleptische Syndrom ist ein seltenes, aber lebensbedrohliches Krankheitsbild, das vor allem durch die Einnahme von Neuroleptika ausgelöst wird und aufgrund seines raschen Verlaufes ebenfalls einen lebensbedrohlichen Notfall darstellt.

Cyclodextrine sind zyklische Oligosaccharide aus α-1,4-glykosidisch verknüpften Glukosemolekülen. ß-Cyclodextrine besitzen sieben Glukoseeinheiten. Bei 2-Hydroxypropyl-β-cyclodextrin (HPBCD) sind die Hydroxygruppen des β-Cyclodextrins (BCD) teilweise mit 2-Hydroxypropylgruppen substituiert. Da der Substituierungsgrad (DS) variieren kann, ist kein einheitliches Molekulargewicht bestimmbar.

Die Erfindung hat erkannt, dass die erfindungsgemäße Formulierung überraschenderweise zu einer signifikant verbesserten Stabilität und Löslichkeit von Dantrolen (bzw. Dantrolen-Natrium) in verschiedenen Medien führt. Eine solche Steigerung der Löslichkeit wird für Komplexe aus Dantrolen oder dessen Salz mit anderen Cyclodextrinen, wie beispielsweise Sulfobutylether-ß-cyclodextrin (SBECD), gerade nicht beobachten. Ein weiterer Vorteil der erfindungsgemäßen Formulierung ist ferner, dass die Konzentration der Wirkstoffkomponente Danrtolen in der wässrigen Formulierung vergleichsweise hoch ist.

Die vorliegende Erfindung ermöglicht es somit, Dantrolen oder dessen Salz mit HPBCD in wässriger oder wasserlöslicher Form als gebrauchsfertige Injektionslösung zur Verfügung zu stellen und damit auf einfacher und schneller Weise einer intravenösen Administration zugänglich zu machen.

Besonders bevorzugt liegt der pH-Wert der Formulierung zwischen 8,5 und 9,5.

Bevorzugt beträgt der Gehalt an HPBCD in der Formulierung 12 bis 40, vorzugsweise 14 bis 30 Gew.-%, weiter vorzugsweise 15 bis 25 Gew.-%.

Weiter bevorzugt ist ein mittlerer Substitutionsgrad (DS) des HPBCDs zwischen 2,5 und 6,3, vorzugsweise zwischen 4 und 5, weiter vorzugsweise 4,6.

In einer bevorzugt Ausführungsform beträgt das Molverhältnis Dantrolen oder dessen Salz:HPBCD 1:1 bis 1:20, vorzugsweise 1:1 bis 1:12.

Besonders bevorzugt ist das Dantrolensalz ein Natriumsalz.

In einer weiteren vorteilhaften Ausführungsform umfasst die Formulierung weitere Komponenten ausgewählt aus der Gruppe aus Verbindungen zum Einstellen des pH-Wertes, Polymeren und Verbindungen zum Modifizieren der Wasserstoffbindungen.

Die erfindungsgemäßen Verbindungen zum Einstellen des pH-Wertes sind bevorzugt ausgewählt aus Puffern, Puffersystemen und/oder Hydoxylsäuren. Besonders bevorzugt sind Natriumhydroxid, Natriumdihydrogenphosphat und/oder Zitronensäure.

Die erfindungsgemäßen Polymere sind bevorzugt ausgewählt aus der Gruppe aus Polyethylenglycolen, Polyvinylpyrrolidonen und Blockcopolymeren aus Ethylenoxid und Propylenoxid. Besonders bevorzugt sind die polymeren Additive PEG300, PVP K-17 und/oder Poloxamer 188.

Die erfindungsgemäßen Verbindungen zum Modifizieren der Wasserstoffbindungen sind bevorzugt ausgewählt aus der Gruppe bestehend aus Tris(hydroxymethyl)aminomethan (TRIS), Ethylendiamintetraessigsäure (EDTA), Glycerol, Arginin, Lysin und Ethanol.

Die Erfindung stellt gemäß einer besonders vorteilhaften Ausführungsform eine zur Injektion geeignete wässrige Lösung zur Verfügung.

Bevorzugt weist die Formulierung eine Konzentration an Dantrolen oder dessen Salz von 0,5 bis 20 mg/ml, vorzugsweise 1 bis 8 mg/ml, weiter vorzugsweise 2 bis 8 mg/ml, am meisten bevorzugt etwa 5 mg/ml, auf.

Gegenstand der Erfindung ist ferner eine gebrauchsfertige Injektionslösung für die intravenöse Administration umfassend die erfindungsgemäße Formulierung.

Insbesondere kann die erfindungsgemäße Formulierung dabei in Form der gebrauchsfertigen Injektionslösung als intravenös zu verabreichendes Notfallmedikament zur Behandlung von Muskelspasmen und Komplikationen im Rahmen der Anwendung von Anästhetika verwendet werden.

Ein weiterer Gegenstand der Erfindung ist ein Verfahren zur Herstellung einer pharmazeutischen Formulierung, gekennzeichnet durch die Schritte:
a)Herstellen einer Mischung von Dantrolen oder dessen Salz und HPBCD,
b) Lösen der Mischung in einer wässrigen Lösung, und
c) gegebenenfalls Einstellen des pH-Wertes der wässrigen Lösung auf einen Wert zwischen 8,5 und 10,5.

Besonders bevorzugt ist es im Rahmen der Erfindung, wenn die hergestellte wässrige Lösung 12 bis 40 Gew.-%, vorzugsweise 14 bis 30 Gew.-%, weiter vorzugsweise 15 bis 25 Gew.-%, des HPBCDs enthält.

Im Rahmen der erfindungsgemäßen Herstellung kann die in Schritt b) und/oder Schritt c) erhaltene Lösung auf 40°C bis 55°C, vorzugsweise etwa 44°C, erhitzt wird.

Besonders bevorzugt wird das Lösen der Mischung über einen Zeitraum von bis zu 24 Stunden erfolgt.

Weiter ist bevorzugt, dass die in Schritt b) und/oder Schritt c) erhaltene Lösung filtriert wird.

Die Erfindung wird nun anhand vorteilhafter Ausführungsformen unter Bezugnahme auf die beigefügten Zeichnungen erläutert. Es zeigt:
- Fig. 1:: Ergebnisse von Löslichkeitsversuchen von Dantrolen-Natrium mit den ß-Cyclodextrinen HPBCD und SBECD.
- Fig. 2:: Analysen der Teilchengröße der Beispielformulierung 2, bestimmt direkt nach der Herstellung (oben) und nach zwei Wochen der Lagerung bei Umgebungstemperatur (unten).

### 1. Eingesetzte Materialien

HPBCD wurde kommerziell von den Firmen Chinoin (DS 2,7), Wacker Chemie (DS 4,6) und Roquette (DS 6,1) erworben.

SBECD wurde als Produkt von der Firma Cyclolab bezogen.

### 2. Untersuchungen der Interaktion zwischen Dantrolen-Natrium und Cyclodextrinen

Untersuchungen zur Phasenlöslichkeit wurden nach Higuchi-Conners in deionisiertem Wasser bei Raumtemperatur nach einer Zeit von 20 Stunden für die Gleichgewichtseinstellung durchgeführt. Nach Einstellung des pH-Wertes (mit Natriumhydroxid, Natriumdihydrogenphosphat, Zitronensäure) folgte erneut eine Zeit von 24 Stunden für die Gleichgewichtseinstellung. Im Anschluss, nach endgültiger pH-Wert-Einstellung (gefolgt von 24 Stunden Gleichgewichtseinstellung), wurden die Konzentrationen des gelösten Dantrolen-Natrium durch UV-Spektrophotometrie bei einer analytischen Wellenlänge von 388 nm, basierend auf einer zuvor registrierten Kalibrierungskurve, bestimmt. Das angewendete UV-spektroskopische Verfahren wurde nicht verifiziert, jedoch vorherige Untersuchungen durchgeführt, um letztendlich den (Matrix-)Effekt der verschiedenen Cyclodextrine auf die UV-spektroskopische analytische Bestimmung von Dantorlen-Natrium zu überprüfen. Diese Untersuchungen zeigten das UV-Spektrophotometrie mit einer Wellenlänge von 388 nm für die zuverlässige und genaue Quantitätsbestimmung von Dantrolen-Natrium in Anwesenheit von Cyclodextrinen in deionisiertem Wasser verwendet werden kann.

### 3. Analyse zur Teilchen-/Aggregatgröße

Die Teilchengröße wurde durch ein Malvern Zetasizer Nano ZS Instrument bestimmt, wobei Verfahren der dynamischen Lichtstreuung verwendet wurden (auch bekannt als Photkorrelationspektroskopie).

### 4. Untersuchungen zur Löslichkeit

Die Untersuchung zur Löslichkeit wurden unter Verwendung von 15 Gew.-%, 20 Gew.-% und 25 Gew.-% HPBCD (HP) sowie 5,0 Gew.-%, 7,0 Gew.-% und 10 Gew.-% SBECD (SB) durchgeführt. Der (endgültige) pH-Wert wurde jeweils auf 7,0, 8,0 und 9,0 eingestellt.

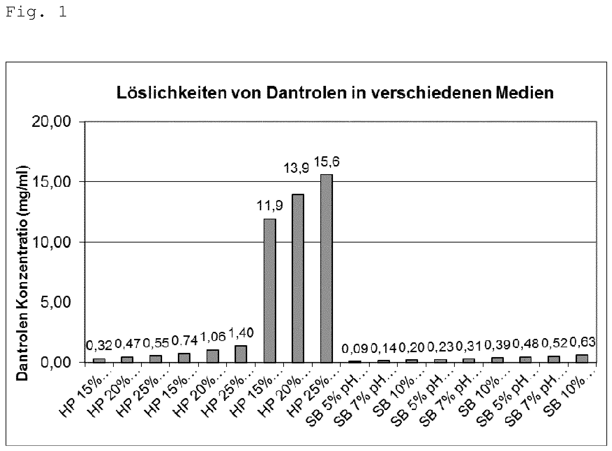

Eine bemerkenswerte Steigerung der Löslichkeit konnte bei einem pH von 9,0 unter Verwendung von HPBCD erzielt werden. Die Ergebnisse der Löslichkeitsversuche sind in Fig. 1 dargestellt.

### 5. Beispiele

Die Probe (25 Gew.-% HPBCD bei pH 9,0), die hinsichtlich der Löslichkeitsuntersuchungen das beste Ergebnis erzielte, wurde zur Herstellung der Beispielformulierungen 1 und 2 ausgewählt. Die Zielkonzentrationen der Formulierungen betrugen 10 mg/ml (etwa zweidrittel Quantität im Vergleich zum gesättigten Wert) und 5 mg/ml.

### Beispiel 1

### Herstellung einer erfindungsgemäßen Formulierung:

In ein 50 ml Gefäß (Glass der hydrolytische Klasse I) wurden 250 mg Dantrolen-Natrium und 6,25 g HPBCD mit einem Substitutionsgrad von 4,6 (auf trockener Basis) eingewogen. Es wurde die dreifache Probenausfertigung hergestellt. Destilliertes Wasser wurde graduell zu der Pulvermischung gegeben, so dass insgesamt 25 ml Flüssigkeit erhalten wurden. Überraschenderweise löste sich die Wirkstoffkomponente (trotz eines vorteilhaften hohen pH von 9,0-9,2) innerhalb von 24 Stunden nicht, erst nach einem leichten Erhitzen (auf 44°C). Beim Erhitzen auf 44°C erfolgte jedoch ein schnelles Lösen. Die klare Lösung wurde durch einen Membranfilter der Firma Agilent mit einer Mikroporengröße von 0,45 filtriert (Klärfiltration).

Erst nach ein paar Tagen der Lagerung bei Raumtemperatur oder im Kühlschrank, begann die Wirkstoffverbindung auszufallen.

### Untersuchungen zur Auswirkung von Verbindungen zum Modifizieren der Wasserstoffbindungen:

Aufgrund der relativen Lagerung der hergestellten Formulierung 1 wurden Löslichkeitsstudien (Higuchi Conners) mit etwaigen zusätzlichen Komponenten(Verbindungen zum Modifizieren der Wasserstoffbindungen), wie TRIS, EDTA, Glycerol, Arginin und Lysin durchgeführt. Der endgültige pH-Wert der Formulierungen wurde auf 9,2 eingestellt. Die Ergebnisse der Löslichkeitstests in Anwesenheit von Verbindungen zum Modifizieren der Wasserstoffbindungen sind in Tab. 1 zusammengefasst.

**Tab. 1: Löslichkeit von Dantrolen in Anwesenheit von Verbindungen zum Modifizieren der Wasserstoffbindungen**

| HPBCD | Zusätze | Gelöstes Dantrolen-Natrium (mg/ml) |
|---|---|---|
| 25 Gew.-% | Keiner | 16,0 |
| 25 Gew.-% | 1 Gew.-% TRIS | 14,4 |
| 25 Gew.-% | 2 Gew.-% Glycerol | 9,5 |
| 25 Gew.-% | EDTA | 3,6 |
| 25 Gew.-% | Arginin | 8,5 |
| 25 Gew.-% | Lysin | 15,7 |

### Untersuchung zur Auswirkung des mittleren Substitutionsgrades (DS) von HPBCD:

Die Löslichkeitstests wurden unter Verwendung von HPBCD mit unterschiedlichem Substitutionsgrad (DS) durchgeführt.

**Tab. 2: Löslichkeit von Dantrolen in Anwesenheit von HPBCD mit verschiedenem Substitutionsgrad (DS)**

| Konzentration von HPBCD | DS | Gelöstes Dantrolen-Natrium (mg/ml) |
|---|---|---|
| 25 Gew.-% | 2.7 | 15.6 |
| 25 Gew.-% | 4.6 | 16.0 |
| 25 Gew.-% | 6.1 | 14.6 |

Die Ergebnisse aus Tab. 2 zeigen, dass ein mittlerer Substitutionsgrad von etwa 4,6 die besten Resultate erzielt.

### Beispiel 2

### Herstellung einer erfindungsgemäßen Formulierung:

In ein 50 ml Gefäß (Glass der hydrolytische Klasse I) wurden 125 mg Dantrolen-Natrium und 6,25 g HPBCD mit einem Substitutionsgrad von 4,6 (auf trockener Basis)eingewogen. Es wurde die zweifache Probenausfertigung hergestellt. Destilliertes Wasser wurde graduell zu der Pulvermischung gegeben, so dass insgesamt 25 ml Flüssigkeit erhalten wurden. Das Lösen wurde durch das Erhitzen der Lösung auf 44°C unterstützt. Die klare Lösung wurde durch einen Membranfilter der Firma Agilent mit einer Mikroporengröße von 0,45 filtriert (Klärfiltration).

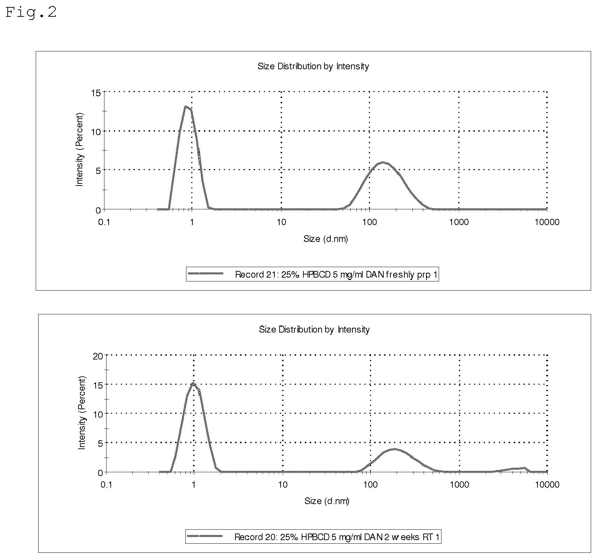

Die resultierende Flüssigkeit war eine klare, dunkelorangefarbende Lösung, die sowohl bei der Lagerung im Kühlschrank als auch bei Umgebungsbedingungen (Temperatur von etwa 24°C), basierend auf einer ersten, visuellen Einschätzung physisch, stabil war. Die Stabilität wurde durch die Analysen der Teilchengröße verfolgt (Photokorrelationsspektroskopie). Die Ergebnisse sind in Fig. 2 dargestellt.

Fig. 2 zeigt die Analysen der Teilchengröße der Beispielformulierung 2, die direkt nach der Herstellung (oben) und nach zwei Wochen der Lagerung bei Umgebungstemperatur (unten) bestimmt wurden. Aus Fig. 2 ist dabei das folgende zu erkennen:
- das Signal bei etwa 1 nm entspricht den nichtaggregierten HPBCD-Fraktionen (zusammen mit den gelösten Wirkstoffmolekülen),
- das Signal bei etwa 200 nm entspricht den aggregierten Fraktionen von HPBCD, die nicht ausschließlich in der Formulierung, sondern auch in der nativen, wässrigen HPBCD-Lösung enthalten sind, und
- das Signal, das bei einer Größe von wenigen Mikrometern während der Lagerung auftritt, deutet auf eine geringe Agglomeration hin.

Die Ergebnisse der Untersuchungen zeigen eine hohe Stabilität der Formulierungen direkt nach der Herstellung, aber auch nach zwei Wochen der Lagerung bei Umgebungstemperatur, obwohl die Proben nicht unter sterilen Bedingungen hergestellt wurden und dadurch eine mikrobiologische Verunreinigung der Formulierungen nicht gänzlich ausgeschlossen werden kann.

### 6. Vorläufige Test zur chemischen Stabilität

Die chemische Stabilität wurde durch Verfolgen der UV-Absorption der Lösungen bei einer analytischen Wellenlänge von 388 nm untersucht. Es wurden drei parallele Messungen durchgeführt. Aus der Beispielformulierung wurden unabhängige Proben in 50 Vol.-% Ethanol hergestellt. Die Konzentrationen an Dantrolen waren die folgenden:
- frisch hergestellte Lösung: 5,16±0,01 mg/ml,
- nach einer Woche der Lagerung (bei Umgebungstemperatur): 5,17±0,04 mg/ml, und
- nach zwei Wochen der Lagerung (bei Umgebungstemperatur): 5,03±0,13 mg/ml (wobei eine sichtbare mikrobiologische Verunreinigung sichtbar wurde).

## Patentansprüche

1. Pharmazeutische Formulierung umfassend einen Komplex aus Dantrolen oder dessen Salz und 2-Hydroxypropyl-β-cyclodextrin (HPBCD), **dadurch gekennzeichnet, dass** der pH-Wert der Formulierung zwischen 8,5 und 10,5 liegt.

2. Formulierung nach Anspruch 1, **dadurch gekennzeichnet, dass** der pH-Wert der Formulierung zwischen 8,5 und 9,5 liegt.

3. Formulierung nach Anspruch 1 oder 2, **dadurch gekennzeichnet, dass** der Gehalt an HPBCD in der Formulierung 12 bis 40, vorzugsweise 14 bis 30 Gew.-%, weiter vorzugsweise 15 bis 25 Gew.-%, beträgt.

4. Formulierung nach einem der Ansprüche 1 bis 3, **dadurch gekennzeichnet, dass** ein mittlerer Substitutionsgrad des HPBCDs zwischen 2,5 und 6,3, vorzugsweise zwischen 4 und 5, weiter vorzugsweise 4,6, ist.

5. Formulierung nach einem der Ansprüche 1 bis 4, **dadurch gekennzeichnet, dass** das Molverhältnis Dantrolen oder dessen Salz:HPBCD 1:1 bis 1:20, vorzugsweise 1:1 bis 1:12, beträgt.

6. Formulierung nach einem der Ansprüche 1 bis 5, **dadurch gekennzeichnet, dass** das Dantrolensalz ein Natriumsalz ist.

7. Formulierung nach einem der Ansprüche 1 bis 6, **dadurch gekennzeichnet, dass** die Formulierung weitere Komponenten ausgewählt aus der Gruppe aus Verbindungen zum Einstellen des pH-Wertes, Polymeren und Verbindungen zum Modifizieren der Wasserstoffbindungen umfasst.

8. Formulierung nach einem der Ansprüche 1 bis 7, **dadurch gekennzeichnet, dass** es eine zur Injektion geeignete wässrige Lösung ist.

9. Formulierung nach einem der Ansprüche 1 bis 8, **dadurch gekennzeichnet, dass** die Formulierung eine Konzentration an Dantrolen oder dessen Salz von 0,5 bis 20 mg/ml, vorzugsweise 1 bis 8 mg/ml, weiter vorzugsweise etwa 5 mg/ml, aufweist.

10. Gebrauchsfertige Injektionslösung für die intravenöse Administration umfassend eine Formulierung nach einem der Ansprüche 1 bis 9.

11. Verfahren zur Herstellung einer Formulierung nach einem der Ansprüche 1 bis 10, **gekennzeichnet durch** die Schritte:
a)Herstellen einer Mischung von Dantrolen oder dessen Salz und HPBCD,
b) Lösen der Mischung in einer wässrigen Lösung, und
c) gegebenenfalls Einstellen des pH-Wertes der wässrigen Lösung auf einen Wert zwischen 8,5 und 10,5.

12. Verfahren nach Anspruch 11, **dadurch gekennzeichnet, dass** die hergestellte Lösung 12 bis 40 Gew.-%, vorzugsweise 14 bis 30 Gew.-%, weiter vorzugsweise 15 bis 25 Gew.-%, des HPBCDs enthält.

13. Verfahren nach Anspruch 11 oder 12, **dadurch gekennzeichnet, dass** die in Schritt b) und/oder Schritt c) erhaltene Lösung auf 40°C bis 55°C, vorzugsweise etwa 44°C, erhitzt wird.

14. Verfahren nach einem der Ansprüche 11 bis 13, **dadurch gekennzeichnet, dass** das Lösen der Mischung über einen Zeitraum von bis zu 24 Stunden erfolgt.

15. Verfahren nach einem der Ansprüche 11 bis 14, **dadurch gekennzeichnet, dass** die in Schritt b) und/oder Schritt c) erhaltene Lösung filtriert wird.
